# EUROPEAN PATENT APPLICATION

(11) **EP 3 991 708 A1**
(43) Date of publication of application: **04.05.2022**
(21) Application number: 20000478.6
(22) Date of filing: 21.12.2020
(51) Int. Cl.: A61G 10/02, A41D 13/002, A61G 13/10

(54) **PROTECTIVE COVER AGAINST VIRUS DROPLET INFECTION**

(30) Priority: 02.11.2020 PL 43584520
(71) Applicant: Politechnika Slaska, 44-100 Gliwice (PL); Buszman, Piotr, 40-761 Katowice (PL)
(72) Inventor: GZIK, Marek, 44-100 Gliwice (PL); BUSZMAN, Piotr, 40-761 Katowice (PL); WOLANSKI, Wojciech, 44-144 Zernica (PL); JOSZKO, Kamil, 42-609 Tarnowskie Góry (PL); GZIK-ZROSKA, Bozena, 44-100 Gliwice (PL); BURKACKI, Michal, 44-100 Gliwice (PL); SUCHON, Slawomir, 44-100 Gliwice (PL); CHRZAN, Milosz, 41-902 Bytom (PL); GRUSZKA, Grzegorz, 40-781 Katowice (PL); PLES, Marek, 43-600 Jaworzno (PL); DOBROWOLSKA, Malgorzata, 40-540 Katowice (PL)

(57) **Abstract**

The present invention relates to a protective cover against virus droplet infection for multiple use in treatment rooms and operating theatres during minimally invasive procedures.

The cover comprises the rigid rectangular base /1/ and the case /2/ with a profile resembling the letter "U" which is fixed to the base /1/ in a dismountable manner. The case /2/ is in the form of the transparent rigid belt/3/ with at least four edges of folds /3'/ and the front transparent apron/ 4/ fixed to the belt /3/ from the front part of the case and on the opposite side the rear transparent apron /5/. Both ends of the rigid belt /3/ are folded outwards creating mounting feet /6/, the base /1/ has along the shorter sides, from the sides of the mounting feet /6/ mounting protrusions /8/ which are folded inwards at an acute angle.

## Description

The present invention relates to a protective cover against virus droplet infection for use in treatment rooms and operating theatres during minimally invasive procedures.

The protective cover against virus droplet infection described in the Chinese utility model no CN2899763 is known. This cover comprises the transparent chamber with a rectangular layout, it is being closed from the front and it has the transparent foil constituting the medical apron which is adjecent to the patient's chest and shoulders after locating the patient's head inside the cover. Inside the chamber the air inlet and outlet connectors are fixed to its walls. The side walls and the upper wall are rigid and they create a profile which reminds the letter "U". The back wall of the chamber is rigid and it aditionally strengthens the whole construction. The walls of the cover are connected with the rectangular base in a dismountable manner by screws.

The protective cover used during treatment of infectious diseases and for limiting their spread is known from the European patent description EP3542658. The protective cover comprises the underpressured cylindric chamber placed on the patient's head. The chamber is equipped from above with the air inlet valve, and on the side wall the connector of the air outlet pipe connected with the air filtration system is fixed. The cover from below is connected tightly with the apron, which enables the patient move his/her head and neck freely.

The patient's protective cover for preventing and treating the epidemic of respiratory system diseases is known from the Chinese patent application description no CN111529260. The cover is used for limiting the spread of biological hazard and it increases the effectiveness of the protection of medical staff. The cover comprises the closed chamber in which the patient's head and shoulders are placed while he/she is lying on the operating table. The chamber is underpressured, and in the chamber's walls there are air inlet and outlet connectors connected with the air pump and with the sterilisation device.

The protective cover for patients suffering from severe acute respiratory syndrome SARS for preventing both medical staff and patients is known from the Chinese patent application description no CN1476941. The cover comprises a cube support whose walls are made of the transparent elastic foil. The chamber of the cover is connected by the air channel with the system of air ventilation and filtration. The patient's bed is placed inside the chamber.

The solution proposed in the present invention aims at designing a reusable protective cover for conscious patients which enables isolation of the patient's upper respiratory system from medical staff during performing medical procedures and it allows fast dismounting of the protective cover in case of the doctor's emergency reactions in the event of the imminent threat to human life.

The present invention relates to a protective cover against virus droplet infection which comprises the chamber with a rectangular base to which the transparent case in the shape resembling the letter "U" equipped with the air inlet and outlet connector and with the elastic apron fixed to it from the front is attached in a dismountable manner, it is characterised by the fact that the case is in the form of the rigid belt with at least four edges of folds, whose both ends are folded outwards creating the mounting feet. The base of the cover has the mounting protrusions along the shorter sides, from the side of the mounting feet. The mounting protrusions are folded towards the inner part of the base at an acute angle. The transparent rear apron constitutes the back wall of the chamber.

It is advantageous when the mounting feet of the case have the notches in the shape corresponding to the shape of the mounting protrusions of the base which create the comb arrangement of mounting.

According to the invention the construction of the protective cover against virus droplet infection
enables the isolation of the patient's head and it allows filtering and eliminating the sources of infection from the exhaled air. As a result of using the protective cover during medical procedures the effectiveness of the protection of medical staff is increased.

The advantage of the construction of the protective cover against virus droplet infection is that by usage of the belt with the edges of folds which rigidify the case of the cover its resilient distorting is possibile, which faciliates assembling and dismantling of the cover.

Thanks to the usage of the comb arrangement of mounting the base to the case of the cover and thanks to the folded mounting protrusions of the base one can achieve the effective immobility of the case in the base which enables their fast assembling and dismounting, and at the same time it enables faster and more effective desinfection of the cover in comparison to the covers which are equipped with slots and mobile connecting parts.

The usage of the transparent apron in the back wall of the protective cover enables easier access to the patient during medical procedures for medical staff.

The described construction of the protective cover enables its reusage during medical procedures, e.g. cardiovascular procedures or the procedures performed in outpatient clinics and other routine procedures.

Thanks to the air inlet and outlet connectors fixed in the walls the protective cover enables connecting with the system of ventilation and filtration of the air in the closed circulation, thanks to which there is an effective protective barrier between the patient and medical staff during performing a procedure, which increases the level of protection against the hazard of the spread of the virus droplet infection.

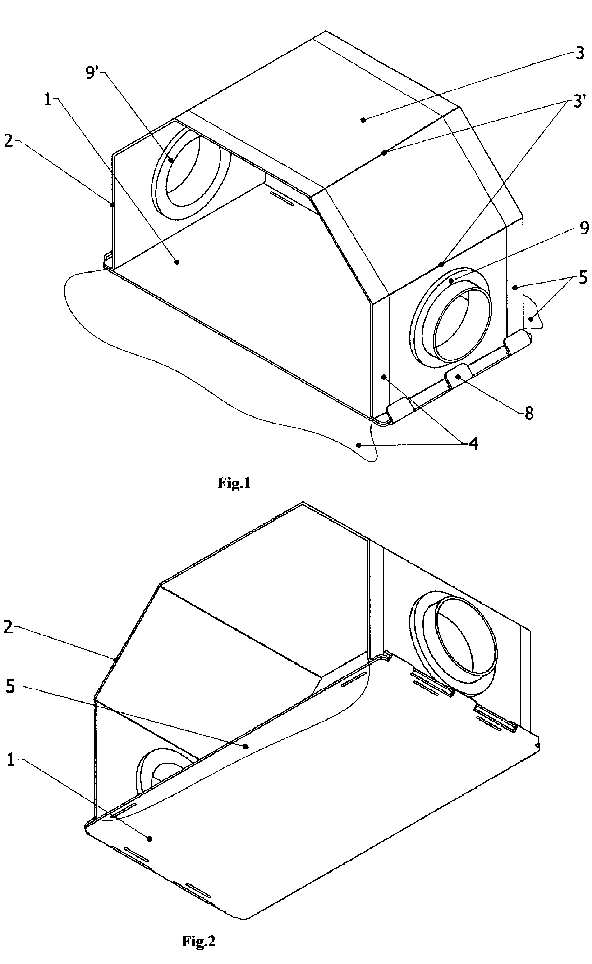

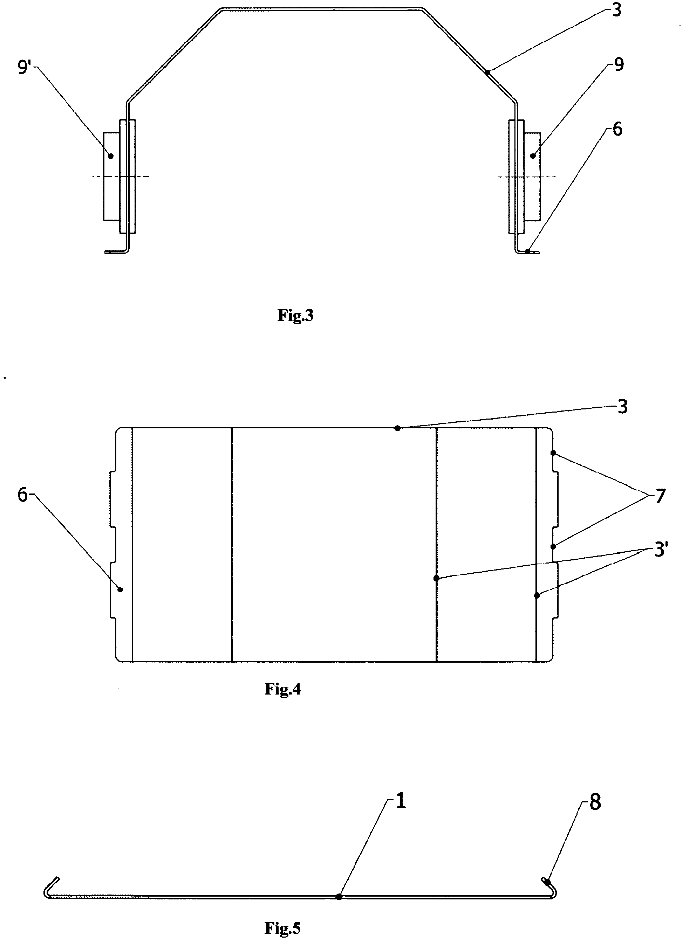

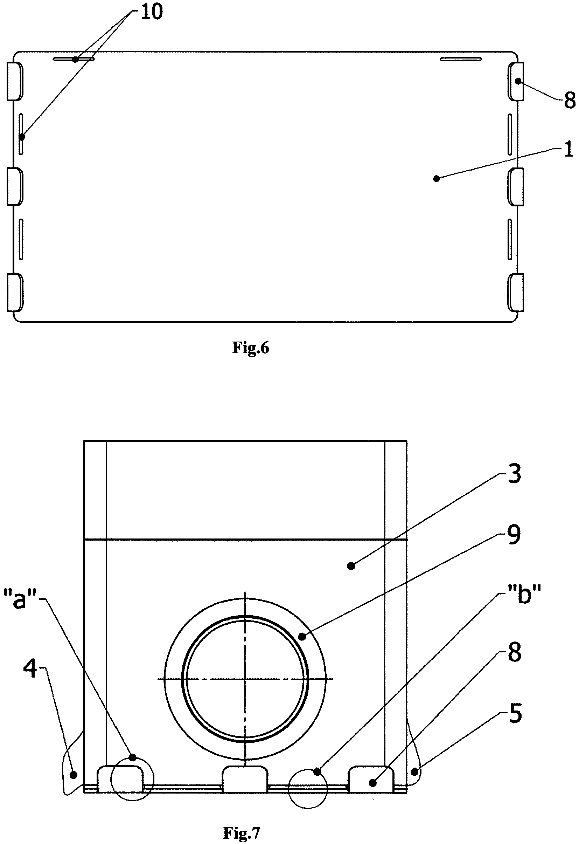

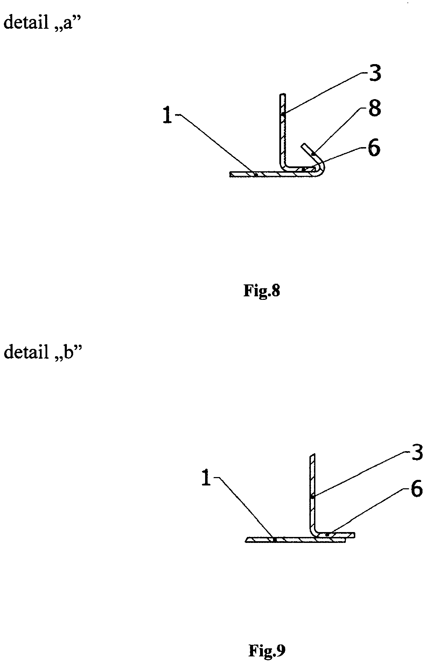

The invention is presented in the examples of realization in the drawings, in which fig. **1** shows the protective cover against virus droplet infection in the axonometric view from the front, fig.**2** - the protective cover in the axonometric view from the back, fig. **3** - the belt of the cover in the view from the front, fig.**4** - the belt of the cover in the view from above without the air inlet and outlet connectors, fig.**5** - the base in the view from the front, fig.**6** the base in the view from above, fig. **7** -the protective cover in the view from the side, fig.**8** - the connection of the belt to the base detail "a", and fig.**9** - -the connection of the belt to the base detail "b".

The protective cover against virus droplet infection is in the form of the chamber with a rectangular base. The cover comprises the rigid rectangular base 1 and the case 2 with a profile resembling the letter "U" which is fixed to the base 1 in a dismountable manner. The case 2 is in the form of the transparent rigid belt 3 with four edges of folds 3' and the transparent front apron 4 fixed to it from the front part of the case 2 in a dismountable manner and on the opposite side the rear transparent apron 5. Both ends of the rigid belt 3 of the case 2 are folded outwards creating mounting feet 6 which have notches 7 in the shape close to a rectangle. The base of the cover 1 has along the shorter sides mounting protrusions 8 in the shape corresponding to the shape of notches 7 in the mounting feet 6. The mounting protrusions 8 are folded towards the inside of the base 1 at an acute angle in order to prevent moving the rigid belt 3 of the case in the vertical direction. In the side walls of the case 2 the air inlet and outlet connectors 9 and 9 ' which enable connecting with the system of ventilation and filtration of the air in the closed circulation are fixed.

The cover is fixed to the tables used during medical procedures by mounting belts which go through the longitudinal protrusions 10 in the base 1.

## Claims

1. The protective cover against virus droplet infection comprises the chamber with the rectangular base to which the transparent case in the shape close to the letter "U" equipped with the air inlet and outlet connector and with the transparent elastic apron fixed to it from the front side is fixed, **charactarised by the fact that** the case is in the form of the rigid belt /3/ with at least four edges of folds /3'/ whose both ends are folded outwards creating mounting feet /6/, and the base /1/ has mounting protrusion /8/ along the shorter sides, from the side of the mounting feet /6/ which are folded inwards at an acute folder, and the rear transparent apron /5/ constitutes the back wall of the chamber.

2. The protective cover of claim 1 **charactarised by the fact that** the mounting feet /6/ of the case /2/ have protrusions /7/ with the shape corresponding to the shape of the mounting protrusions /8/ of the base.
